# EUROPEAN PATENT SPECIFICATION

(11) **EP 2 364 123 B1**
(45) Date of publication and mention of the grant of the patent: **28.12.2016**
(21) Application number: 09747941.4
(22) Date of filing: 26.10.2009
(51) Int. Cl.: A61C 5/06, A61M 5/00, A61M 5/315, A61M 5/36

(54) **CONTAINER FOR STORING AND DISPENSING A FLOWABLE MATERIAL**
BEHÄLTER ZUR AUFBEWAHRUNG UND AUSGABE EINES FLIESSFÄHIGEN MATERIALS
RÉCIPIENT POUR STOCKER ET DISTRIBUER UNE MATIÈRE LIQUIDE

(30) Priority: 24.10.2008 US 197194 P
(43) Date of publication of application: 14.09.2011
(73) Proprietor: DENTSPLY International Inc., York, PA 17405-0872 (US)
(72) Inventor: SIRKIS, James, Ronald, Milford, DE 19963 (US)
(74) Representative: Blodig, Wolfgang
(86) International application number: PCT/US2009/005823
(87) International publication number: WO 2010/047840

(56) References cited:
- WO-A-2006/007592
- WO-A-2006/110023
- WO-A-2009/077091
- DE-A1-102004 055 870
- US-A- 5 971 953

## Description

### Technical Field

The present invention is a container for the storage and dispensing of a flowable material. More particularly, the invention is such a package having an inlet end, an applicator or dispensing end and a movable piston or plug therein. The piston is at least partially transmissive of at least one medium such as air, water, contaminant or other component, such as disclosed in WO2006/110023 and WO2006/007592.

### Background of the Invention

Containers or packages for the storing and dispensing of flowable materials are known in many arts. By "flowable" or similar words used herein it is meant any material that can be expressed by being directed toward or physically pushed toward a discharge end of a container. The material may have low viscosity like water or high viscosity resistant to flow. In the latter case, application of heat, vibration or other energy is often required to affect a flow of the material. All such materials are useful within the concepts of the present invention.

For example, U.S. Pat. No. 5,707,234 shows a container in the form of a cartridge that is particularly suited for use with dental materials. The cartridge has a container body or capsule, an outlet end or discharge nipple and an inlet end. After the material is placed within the container body normally through the inlet end, a piston or plug closes the inlet end. The piston is movable within the container body such that as a mechanical force is applied to the piston, it moves toward the discharge end. The material is thus caused to move toward the outlet end to be dispensed.

It has been found that in some cases when such a container is filled and the plug is inserted, not all of the air is removed from between the plug and the container's fill level. This trapped air in the package has a spring like quality. That is, as the plug is advanced during expression of the product the air compresses and when the plug stops moving the air expands causing more material to express from the container. This action has the undesired effect of causing the end user to get inconsistent expression of material; i.e., material continues to flow when the user wants it to stop flowing. This concept of undesired and unwanted flow of material out of the nozzle is called ooze.

Ooze prevents precise application of the contained product or material and is unacceptable to the end user. Additionally trapped air in a package can cause leakage of product due to pressure differentials between the contents and the surrounding environment during storage and transportation. Air trapped in the package may mix with the product during expression of the material and produce voids in the product, possibly compromising the quality of the end result.

A need exists therefore for a storage and dispensing package that has the useful storage and dispensing qualities of a container such as in the '234 patent, but which is able to self-vent and compensate for air trapped within the container during the filling or other procedures.

### Summary of the Invention

In general, a package for storing and dispensing a flowable dental material as disclosed in claim 1 is the present invention.

### Brief Description of the Drawings

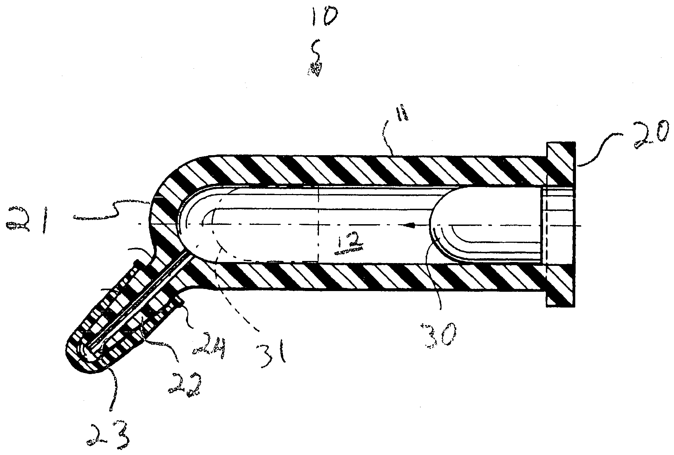

Fig. 1 is a side elevation, sectional view of a package according to the concepts of the present invention.

### Preferred Embodiments for Carrying Out the Invention

A package for the storage and dispensing of a flowable material according to the concepts of the present invention is generally designated by the number 10 on the attached drawing. Package 10 has a container body 11 configured to have an open area or cavity 12 therein for storing a flowable material (not shown). Package 10 has an inlet end 20 and an outlet end 21. Although not necessarily a part of the present invention, one preferred package 10 has an outlet end 21 fitted with a discharge nipple 22 having a passage 23 therethrough and in fluid communication between cavity 12 and the atmosphere. Discharge nipple 22 is fitted with a removable cap 24 to selectively close off passage 23.

A preselected quantity of flowable material (or as discussed above, a material capable of being induced to flow by any means) such as a dental adhesive or restorative is placed into cavity 12 by standard manufacturing techniques that are well known in the industrial arts, and preferably through inlet end 20. A movable piston or plug 30 is then received within inlet end 20 such that piston 30 closes off, and at least selectively seals inlet end 20. Piston 30 is moveable within cavity 12 such as is shown by phantom lines 31, and preferably in a direction toward outlet end 21. In this manner, the material (not shown) is caused to move toward outlet end 21 to be discharged therefrom, such as by passing through passage 23 and with cap 24 removed, out to the atmosphere. As alluded to above, any mechanical force may be applied to piston 30 to effect such movement. The specific means of inducing such force is well known in the art and includes for examples guns and syringes. All such means are within the scope of the invention and the particular means is not germane to the invention as long as some means is provided to move piston 30.

As discussed above, during the filling or other procedures, a quantity of air or other contaminant may be trapped within cavity 12 along with the desired material. For example, carriers such as water, alcohols or the like may be present with or even leach from the material itself and it may be desirable to remove these mediums. According to the present invention piston 30 is at least partially permeable to one or more such mediums such that the selected medium can be caused to pass through piston 30 to thereby be removed from package 10, but not permeable to the material to be dispensed. In this manner, either during the filling stage or even in use by the end user, the undesired or selected medium can be caused to flow through piston 30 and out of package 10 while the material to be dispensed is moved along by the action of piston 30 as was above described.

The specific material from which a piston 30 will be fabricated will vary according to the material itself. For example, pistons may be fabricated from silicones, epoxy, rubber, glass, ceramics, porcelains and mixtures thereof. Useful polymeric materials include for example, polyethylene (PE), polypropylene (PP), polyamines such as nylon-6, polytetrafluouroethylene (PTFE), ultra-high molecular weight polyethylene (UHMWPE), high-density Polyethylene (HDPE), polyvinylidene fluoride (PVDF), ethyl vinyl acetate (EVA), thermoplastic polyurethane (TPU), polyester (PET), low-density polyethylene (LDPE) and mixtures thereof. Piston 30 may be fabricated from a pliable material, a resilient material, a soft or even a hard material, again based upon the specific needs of a given material to be stored and dispensed. A woven fabric or mesh, crystals, tubes, granules, irregular shaped materials and the like are within the scope of the invention. In a preferred embodiment, piston 30 will provide a seal between the outside atmosphere and the material stored within cavity 12.

It is also within the scope of the invention to provide a piston 30 fabricated from a single material of selected porosity or to fabricate a piston 30 having only a portion or selected portions permeable by or porous to a selected or indeed a plurality of selected mediums. For example, one portion of piston 30 may be fabricated from a stronger material for strength or a pliable material for its sealing effect, while one or more other portions are porous to or permeable by the selected medium or mediums. According to the present invention, the piston 30 has one portion permeable by air, another permeable by water yet no portion permeable by the material to be stored or dispensed. It is also within the scope of the invention to provide a piston 30 that is selectively permeable to a medium at a given rate. For example, in certain applications a piston 30 may be permeable to a selected medium but only over time. By properly selecting the material from which to fabricate such a piston 30, its pore sizes, shapes or the like, the medium can be caused to permeate piston 30 at a preselected rate.

It is also within the scope of the invention to provide for other components in the alternative to or in addition to the piston which likewise are permeable to the selected medium, such as cap 24 for example. The invention has been described with respect to the piston 30 being the means by which selected porosity or permeability to a medium is achieved, but that is a preferred embodiment only. Other components of package 10 may be so configured. Similarly, the invention has been described herein for exemplary purposes as being useful for the removal or egress of a medium from package 10. It is equally within the scope of the invention to provide a package 10 as described herein that permits the ingress of a medium into cavity 10. All such configurations are within the scope of the present invention.

It is evident therefore that package as described herein provides an advantageous contribution to the art. The invention has been described and shown on the drawings for exemplary purposes only and without attempting to show each and every variation within the scope thereof. The scope of the invention shall only be determined by any attached claims.

## Claims

1. A package (10) for storing and dispensing a flowable dental material selected from a dental adhesive or restorative, said package (10) being a cartridge comprising a container body (11) having an inlet end (20) and an outlet end (21); said container body (11) holding a quantity of the material in a cavity (12), said outlet end (21) fitted with a discharge nipple (22) having a passage (23) therethrough and in fluid communication between the cavity (12) and the atmosphere; said discharge nipple (22) fitted with a removable cap (24) to selectively close off passage (23); and having a piston (30) receivable within said inlet end (20) and movable within said container body (11); **characterized in that** at least a portion of said piston (30) is permeable to air,
another portion permeable to water, and no portion is permeable to the flowable dental material.

2. A package (10) as in claim 1 wherein said piston (30) is fabricated from a material selected from the group consisting of polyethylene (PE), polypropylene (PP), polyamines, polytetrafluouroethylene (PTFE), ultra-high molecular weight polyethylene (UHMWPE), high-density Polyethylene (HDPE), polyvinylidene fluoride (PVDF), ethyl vinyl acetate (EVA), thermoplastic polyurethane (TPU), polyester (PET), low-density polyethylene (LDPE) and mixtures thereof.

3. A package (10) as in claim 1 wherein said piston (30) is fabricated from a material selected from the group consisting of silicones, epoxy, rubber, glass, ceramics, porcelains and mixtures thereof.

4. A package (10) as in claim 1 wherein said piston (30) is a woven fabric.

## Patentansprüche

1. Behälter (10) zum Lagern und Ausgeben eines fließfähigen Dentalmaterials, ausgewählt aus einem dentalen Adhäsiv oder einem restaurativen Mittel, wobei der Behälter (10) eine Kartusche ist, die einen Behälterkörper (11) mit einem Einlassende (20) und einem Auslassende (21) aufweist; wobei der Behälterkörper (11) eine Menge des Materials in einem Hohlraum (12) trägt; wobei an dem Auslassende (21) ein Auslassstutzen (22) angebracht ist, welcher einen Durchgang (23) aufweist und in Fluidverbindung zwischen dem Hohlraum (12) und der Umgebung steht; wobei an dem Auslassstutzen (22) ein abnehmbarer Verschluss (24) angebracht ist, um den Durchgang (23) selektiv zu verschließen; und einen Kolben (30) aufweist, der in dem Einlassende (20) aufgenommen werden kann und in dem Behälterkörper (11) beweglich ist; **dadurch gekennzeichnet, dass** wenigstens ein Teil des Kolbens (30) für Luft durchlässig ist, ein anderer Anteil für Wasser durchlässig ist und kein Anteil für das fließfähige Dentalmaterial durchlässig ist.

2. Behälter (10) nach Anspruch 1, wobei der Kolben (30) aus einem Material hergestellt ist,. ausgewählt aus der Gruppe, bestehend aus Polyethylen (PE), Polypropylen (PP), Polyaminen, Polytetrafluorethylen (PTFE), ultrahochmolekularem Polyethylen (UHMWPE), Polyethylen mit hoher Dichte (HDPE), Polyvinylidenfluorid (PVDF), Ethylvinylacetat (EVA), thermoplastischem Polyurethan (TPU), Polyester (PET), Polyethylen mit geringer Dichte (LDPE) und Mischungen derselben.

3. Behälter (10) nach Anspruch 1, wobei der Kolben (30) aus einem Material hergestellt ist, ausgewählt aus der Gruppe, bestehend aus Silikonen, Epoxid, Gummi, Glas, Keramiken, Porzellanen und Mischungen derselben.

4. Behälter (10) nach Anspruch 1, wobei der Kolben (30) ein Fasergewebe ist.

## Revendications

1. Emballage (10) pour stocker et distribuer un matériau dentaire fluide choisi entre un adhésif ou un matériau de restauration dentaire, ledit emballage (10) étant une cartouche comprenant un corps de récipient (11) ayant une extrémité d'entrée (20) et une extrémité de sortie (21) ; ledit corps de récipient (11) maintenant une quantité du matériau dans une cavité (12), ladite extrémité de sortie (21) étant équipée d'un manchon de décharge (22) ayant un passage (23) à travers celui-ci et en communication fluidique entre la cavité (12) et l'atmosphère ; ledit manchon de décharge (22) étant équipé d'un capuchon amovible (24) pour fermer de manière sélective le passage (23) ; et ayant un piston (30) pouvant être reçu à l'intérieur de ladite extrémité d'entrée (20) et mobile à l'intérieur dudit corps de récipient (11) ;
**caractérisé en ce qu'**au moins une partie dudit piston (30) est perméable à l'air, une autre partie est perméable à l'eau et aucune partie n'est perméable au matériau dentaire fluide.

2. Emballage (10) selon la revendication 1, dans lequel ledit piston (30) est fabriqué à partir d'un matériau choisi dans le groupe consistant en le polyéthylène (PE), le polypropylène (PP), des polyamines, le polytétrafluoroéthylène (PTFE), le polyéthylène de poids moléculaire ultra-élevé (UHMWPE), le polyéthylène haute densité (HDPE), le polyfluorure de vinylidène (PVDF), l'éthylène-acétate de vinyle (EVA), le polyuréthane thermoplastique (TPU), le polyester (PET), le polyéthylène basse densité (LDPE) et leurs mélanges.

3. Emballage (10) selon la revendication 1, dans lequel ledit piston (30) est fabriqué à partir d'un matériau choisi dans le groupe consistant en les silicones, l'époxy, le caoutchouc, le verre, la céramique, la porcelaine et leurs mélanges.

4. Emballage (10) selon la revendication 1, dans lequel ledit piston (30) est un tissu tissé.
